(19) Europäisches Patentamt
European Patent Office
Office européen des brevets

(11) Veröffentlichungsnummer: **0 075 205**
**A2**

# (12) EUROPÄISCHE PATENTANMELDUNG

(21) Anmeldenummer: **82108290.6**

(22) Anmeldetag: **09.09.82**

(51) Int. Cl.³: **C 07 C 157/14**, C 07 D 261/08, C 07 D 271/06, A 01 N 47/42

(30) Priorität: **17.09.81 DE 3136891**

(43) Veröffentlichungstag der Anmeldung: **30.03.83 Patentblatt 83/13**

(84) Benannte Vertragsstaaten: **AT BE CH DE FR GB IT LI NL SE**

(71) Anmelder: **BASF Aktiengesellschaft, Carl-Bosch-Strasse 38, D-6700 Ludwigshafen (DE)**

(72) Erfinder: **Acker, Rolf-Dieter, Dr., Tuchbleiche 8, D-6906 Leimen (DE)**
Erfinder: **Theobald, Hans, Dr., Parkstrasse 2, D-6703 Limburgerhof (DE)**
Erfinder: **Wuerzer, Bruno, Dr. Dipl.-Landwirt, Ruedigerstrasse 13, D-6701 Otterstadt (DE)**

## (54) Isothioharnstoffe, Verfahren zu ihrer Herstellung und ihre Verwendung zur Bekämpfung unerwünschten Pflanzenwuchses.

(57) Die Erfindung betrifft Isothioharnstoffe der Formel

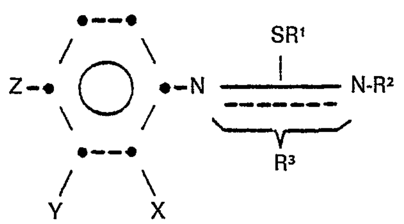

(I)

worin $R^1$, $R^2$, $R^3$, X, Y und Z die in der Beschreibung angegebenen Bedeutungen haben, und deren Salze. Weiter betrifft die Erfindung die Herstellung dieser Substanzen und deren Verwendung zur Bekämpfung unerwünschten Pflanzenwuchses.

EP 0 075 205 A2

Isothioharnstoffe, Verfahren zu ihrer Herstellung und ihre Verwendung zur Bekämpfung unerwünschten Pflanzenwuchses

Die Erfindung betrifft neue Isothioharnstoffe, deren Herstellung, Herbizide, die diese Verbindungen als Wirkstoffe enthalten, und deren Verwendung bei der Bekämpfung unerwünschten Pflanzenwuchses.

N,N-Dimethylisothioharnstoffe sind als Akarizide und Lepidopterizide bekannt (US-PS 3 992 447, US-PS 4 049 826). Auch herbizide Wirkungen sind von Verbindungen dieser Klasse bekannt (DE-OS 16 43 809, NL-OS 67 13 501, GB-PS 945 808). Analoges gilt für eine algizide Wirkung (FR-PS 1 524 675). Auch bei O-alkylierten Harnstoffen und auch Isothioharnstoffen wurde eine herbizide Wirkung beobachtet (US-PS 2 849 306).

Es wurde nun gefunden, daß Isothioharnstoffe der Formel I

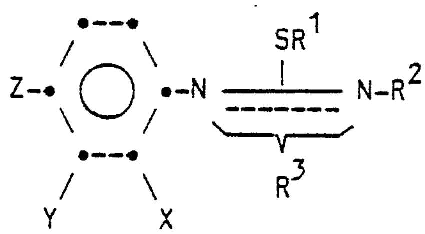 (I)

und ihre Salze,
wobei die Formel I die Isomeren der Formel Ia

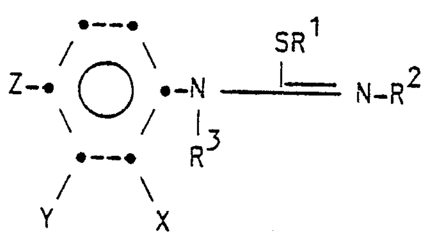 (Ia)

und der Formel Ib

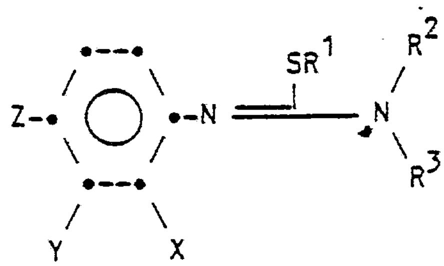

(Ib)

kennzeichnet, worin X, Y und Z gleich oder verschieden sind und Wasserstoff, Halogen, $C_{1-4}$-Alkyl, $C_{1-4}$-Alkoxy, $C_{1-4}$-Halogenalkyl, $C_{1-4}$Halogenalkoxy, $C_{1-4}$-Alkylthio,
$R^1$ $C_{1-7}$-Alkyl oder $C_{3-7}$-Cycloalkyl, $C_{1-4}$-Halogenalkyl, $C_{2-8}$-Alkylthioalkyl, Benzyl, substituiertes Benzyl, $C_{2-4}$-Alkenyl, $C_{2-4}$-Halogenalkenyl, $C_{2-4}$-Alkinyl, einen heterocyclischen Rest tragendes $C_1$-$C_4$-Alkyl, Aryl-oxy-$C_{1-4}$-alkyl,
$R^2$ Wasserstoff, $C_{1-7}$-Alkyl oder $C_{3-7}$-Cycloalkyl,
$R^3$ Wasserstoff oder die Gruppe -CO-OR$^4$, worin $R^4$ $C_{1-3}$Alkyl ist,
bedeuten, mit der Maßgabe, daß wenn Z Chlor, Y Wasserstoff, X Wasserstoff, $R^2$ Alkyl und $R^3$ Wasserstoff sind, $R^1$ nicht Alkyl ist und $R^2$ und $R^3$ nicht gleichzeitig Wasserstoff bedeuten, eine beachtliche selektive herbizide Wirkung besitzen.

Als besonders wirksam haben sich die Verbindungen erwiesen, in denen X Wasserstoff, Y Wasserstoff, Fluor oder Chlor, Z Wasserstoff oder Chlor, $R^1$ eine durch einen gegebenenfalls methylierten Isoxazolyl-, Thiadiazolyl- oder Oxadiazolylrest oder einen gegebenenfalls chlorierten Phenyl- oder Trichlorethylenrest substituierte Methylgruppen, $R^2$ eine Methyl- oder Acetoxygruppe und $R^3$ Wasserstoff darstellen. Für $R^1$ ist insbesondere der 3-Methyl-isooxazol-5-yl-methylrest zu nennen.

Die neuen Verbindungen lassen sich herstellen, indem man

a) eine Halogenalkyl-Verbindung der Formel

$$Hal-R^1 \quad (II),$$

worin $R^1$ die oben angegebenen Bedeutungen hat und Hal Halogen bedeutet, mit einem Thioharnstoff der Formel

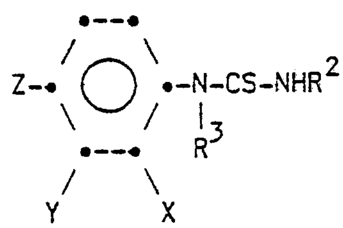 (III),

worin X, Y, Z, $R^2$ und $R^3$ die obengenannten Bedeutungen haben, oder

b) einen Isothioharnstoff der Formel

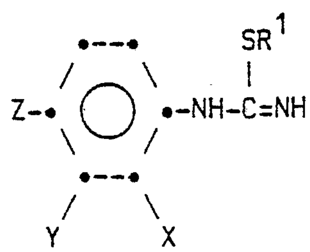 (IV),

worin X, Y, Z und $R^1$ die obengenannten Bedeutungen haben, mit einem Chlorameisensäureester der Formel

$$Hal-CO-OR^4 \quad (V),$$

worin $R^4$ die oben angegebenen Bedeutungen hat und Hal für Halogen steht, umsetzt und die so erhaltenen Verbindungen gegebenenfalls anschließend in ihre Salze überführt.

Die Reaktion a) gelingt bei einer Temperatur von 0 bis 150°C. Zweckmäßigerweise arbeitet man in Gegenwart einer Base. Setzt man keine Base zu, so entsteht das Salz, aus dem die Base anschließend isoliert werden kann.

Das Verfahren b) wird mit ungefähr stöchiometrischen Substanzmengen, d.h. in einem Mengenverhältnis von etwa 0,8 bis 1,2 Verbindung IV zu 1,0 Verbindung V, durchgeführt. Zweckmäßigerweise werden die Verbindung IV in einem inerten Lösungsmittel vorgelegt und die Base und deren Chlorameisensäureester unter Rühren bei einer Temperatur von -10 bis +80°C, vorzugsweise 10 bis 50°C zugegeben. Zur Vervollständigung der Reaktion rührt man noch 15 Minuten bis 24 Stunden, vorzugsweise 1 Stunde bis 5 Stunden, und filtriert vom Ungelösten ab oder - je nach Verwendung der Base - trennt die Phasen, trocknet und engt das organische Lösungsmittel ein. Die Reinigung erfolgt durch Umkristallisieren oder Chromatographie.

Die Isothioharnstoffe der Formel IV liegen entsprechend den Verbindungen der Formel I als Gleichgewichtsmischung vor (vgl. Formeln Ia und Ib).

Beide Verfahren können kontinuierlich oder diskontinuierlich, drucklos oder unter Druck, durchgeführt werden; der Einfachheit halber wird Atmosphärendruck bevorzugt.

Die Umsetzungen werden in unter den Reaktionsbedingungen inerten Lösungsmitteln durchgeführt. Hierfür kommen z.B. in Betracht: Wasser; Alkohole, wie z.B. Methanol, Ethanol, n-Propanol, Isopropanol, n-Butanol, s-Butanol, t-Butanol; Halogenkohlenwasserstoffe, insbesondere Chlorkohlenwasserstoffe, z.B. Tetrachlorethylen, 1,1,2,2- oder 1,1,1,2-Tetrachlorethan, Dichlorpropan, Methylenchlorid, Dichlorbutan,

Chloroform, Chlornaphthalin, Dichlornaphthalin, Tetrachlorkohlenstoff, 1,1,1- oder 1,1,2-Trichlorethan, Trichlorethylen, Pentachlorethan, o-, m-mp-Difluorbenzol, 1,2-Dichlorethan, 1,1-Dichlorethan, 1,2-cis-Dichlorethylen, Chlorbenzol, Fluorbenzol, Brombenzol, Iodbenzol, o-, p- und m-Dichlorbenzol, o-, p-, m-Dibrombenzol, o-, m-, p-Chlortoluol, 1,2,4-Trichlorbenzol; Ether, z.B. Ethylpropylether, Methyl-tert.-butylether, n-Butylethylether, Di-n--butylether, Diisobutylether, Diisoamylether, Diisopropylether, Anisol, Phenetol, Cyclohexylmethylether, Diethylether, Ethylenglykoldimethylether, Tetrahydrofuran, Dixoan, Thioanisol, ß,ß'-Dichlordiethylether; Nitrokohlenwasserstoffe, wie Nitromethan, Nitroethan, Nitrobenzol, o-, m-, p-Chlornitriobenzol, o-Nitrotoluol; Nitrile, wie Acetonitril, Butyronitril, Isobutyronitril, Benzonitril, m-Chlorbenzonitril; aliphatische oder cycloaliphatische Kohlenwasserstoffe, z.B. Heptan, Pinan, Nonan, o-, m-, p-Cymol, Benzinfraktionen innerhalb eines Siedepunktintervalls von 70 bis 190°C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; Ester, z.B. Ethylacetat, Acetessigester, Isobutylacetat; Amide, z.B. Formamid, Methylformamid, Dimethylformamid; Ketone, z.B. Aceton, Methylethylketon; und entsprechende Gemische. Zweckmäßig verwendet man das Lösungsmittel in einer Menge von 100 bis 2 000 Gew.%, vorzugsweise von 200 bis 700 Gew.%, bezogen auf die Ausgangsstoffe.

Als Basen kommen alle üblichen Säurebindemittel in Frage. Hierzu gehören vorzugsweise tertiäre Amine, Erdalkaliverbindungen, Ammoniumverbindungen und Alkaliverbindungen sowie entsprechende Gemische. Es können aber auch Zinkverbindungen verwendet werden. Beispiele hierfür sind: Kaliumhydroxid, Natriumhydroxid, Kaliumcarbonat, Natriumcarbonat, Lithiumhydroxid, Lithiumcarbonat, Natriumhydrogencarbonat,

Kaliumhydrogencarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumhydrogencarbonat, Magnesiumacetat, Zinkhydroxid, Zinkoxid, Zinkcarbonat, Zinkacetat, Natriumformiat, Natriumacetat, Trimethylamin, Triethylamin, Tripropylamin, Triisopropylamin, Tributylamin, Triisobutylamin, Tri-sec-butylamin, Tri-tert.-butylamin, Tribenzylamin, Tricyclohexylamin, Triamylamin, Diisopropylethylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-p-aminopyridin, N,N-Diethyl-p-aminopyridin, N,N-Dipropyl-p-aminopyridin, N-Methylpyrrolidon, N-Ethylpyrrolidon, N-Methylpiperidin, N-Ethylpiperidin, N-Methylpyrrolidin, N-Ethylpyrrolidin, N-Methylimidazol, N-Ethylimidazol, N-Methylpyrrol, N-Ethylpyrrol, N-Methylmorpholin, N-Ethylmorpholin, N-Methylhexamethylenimin, N-Ethylhexamethylenimin, Pyridin, Chinolin, α-Picolin, ß-Picolin, γ-Picolin, Isochinolin, Pyrimidin, Acridin, N,N,N',N'-Tetramethylethylendiamin, N,N,N',N'-Tetraethylethylendiamin, Chinoxalin, Chinazolin, N-Propyldiisopropylamin, N,N'-Dimethylcyclohexylamin, 2,6-Lutidin, 2,4-Lutidin, Trifurylamin, Triethylendiamin.

Außer den vorgenannten anorganischen Säurebindemitteln kommen ferner z.B. Natriumpropionat, Natriumbutyrat, Natriumisobutyrat, Kaliumformiat, Kaliumacetat, Kaliumpropionat, Kaliumbutyrat, Kaliumisobutyrat, Natriummethylat, Natriumethylat, Natriumpropylat, Natriumisopropylat, Natriumbutylat, Natriumisobutylat, Natrium-sec-butylat, Natrium-tert.-butylat, Natriumethylenglykolat, Natriumpropylen-(1,2)-glykolat, Natriumpropylen-(1,3)-glykolat, Natriumdiethylenglykolat, Natriumtriethylenglykolat, Natriumdipropylen-(1,2)-glykolat, Kaliummethylat, Kaliumethylat, Kalium-n-propylat, Kaliumisopropylat, Kalium-n--butylat, Kalium-isobutylat, Kalium-sec-butylat, Kalium-tert.-butylat, Kaliummethylenglykolat, Kaliumpropylen-(1,2)-glykolat, Kaliumpropylen-(1,3)-glykolat, Kaliumdiethylenglykolat, Kaliumtriethylenglykolat, Kaliumdipropylen-(1,2)-glykolat in Frage.

Die Isothioharnstoffe der Formel I können nach bekannten Verfahren in ihre für Nutzpflanzen verträglichen Salze überführt werden. Zur Salzbildung eignen sich beispielsweise Halogenwasserstoffsäuren (HCl, HBr, HJ), Schwefelsäure, Salpetersäure, Phosphorsäure, Essigsäure, Dodecylbenzolsulfonsäure, Ameisensäure, Oxalsäure und Phthalsäure.

Die Wirksamkeit der Salze geht auf das Kation zurück, so daß die Wahl des Anions beliebig ist, sofern das Anion keine Schäden hervorruft.

Die folgenden Beispiele erläutern die Herstellung der Verbindungen der Formel I. Die angegebenen Teile sind Gewichtsteile.

Beispiel 1

a) 76 Teile Phenylthioharnstoff, 855 Teile Benzylbromid und 100 Teile Ethanol werden zusammengegeben und 1 h unter Rückfluß erhitzt. Nach dem Abkühlen wird eingeengt, man erhält 161 Teile 1-Phenyl-2-benzyl-isothioharnstoff als Hydrobromid vom Fp. 128 bis 130°C.

b) 160 Teile der so erhaltenen Verbindung werden mit 300 Teilen Wasser versetzt. Man fügt solange gesättigte Natriumcarbonat-Lösung zu, bis pH = 9 erreicht ist. Der kristalline Rückstand wird abgesaugt und getrocknet. Man erhält 115 Teile 1-Phenyl-2-benzyl-isothioharnstoff vom Fp. 75 bis 77°C.

c) Die gemäß b) erhaltene Verbindung läßt sich wie folgt darstellen: 76 Teile Phenylthioharnstoff, 85,5 Teile Benzylbromid werden in 300 Teilen Dichlorethan vorgelegt. Man läßt bei 10°C 22 Teile Natriumethanolat in 70 Teilen Methanol zulaufen und rührt 3 h bei 30 bis 40°C nach. Die Mischung wird filtriert, das Lösungsmittel abdestilliert, der Rückstand in Methylenchlorid aufgenommen und mit Wasser gewaschen. Nach dem Trocknen und Einengen erhält man 118 Teile 1-Phenyl-2-benzyl-isothioharnstoff vom Fp. 74 bis 76°C.

15 Teile 1-Phenyl-2-benzyl-isothioharnstoff werden in 50 Teilen Methylenchlorid vorgelegt. Man fügt gleichzeitig 6,6 Teile Chlorameisensäureester und eine Lösung von 2,8 Teilen Natriumhydrid in 7 Teilen Wasser portionsweise zu und hält die Reaktionstemperatur bei 10 bis 15°C. Man rührt 3 h bei Raumtemperatur, trennt die organische Phase ab und wäscht mit Wasser nach. Nach dem Trocknen mit Natriumsulfat wird die organische Lösung eingeengt und der Rückstand aus Methanol umkristallisiert.

Man erhält 14,2 Teile 1-Phenyl-3-carboxymethyl-2-benzyl-isothioharnstoff vom Fp. 98 bis 99°C.

Beispiel 2

a) 22,1 Teile 1-(4-Chlorphenyl)-3-methyl-thioharnstoff, 14,5 Teile 3-Methyl-2-chlormethylisoxazol und 30 Teile Ethanol werden 5 h unter Rückfluß erhitzt. Nach dem Abkühlen wird die Lösung eingeengt und der Rückstand an der Ölpumpe getrocknet. Man erhält 36,1 Teile des Hydrochlorids vom 1-(4-Chlorphenyl)-2-(3-methyl--isoxazol-5-yl-methyl)-3-methyl-isothioharnstoff vom Fp. 151 bis 154°C.

b) 29 Teile des so erhaltenen Salzes werden in Wasser gelöst. Danach wird gesättigte Natriumcarbonatlösung bis zum Erreichen von pH = 9 zugefügt. Nach 15 min Rühren wird abgesaugt und der Rückstand getrocknet. Man erhält 22,8 Teile 1-(4-Chlorphenyl)-2-(3-methyl--isoxazol-5-yl-methyl)-3-methyl-isothioharnstoff vom Fp. 100 bis 103°C.

Analaog Beispiel 1 und 2 wurden folgende Verbindungen der Formel Ia hergestellt, wobei zu beachten ist, daß diese Verbindungen im Fall $R^3$ = H mit der Formel Ib im Gleichgewicht stehen.

In der Tabelle werden folgende Abkürzungen verwendet:

A: 2,6-Dichlorbenzyl

B: 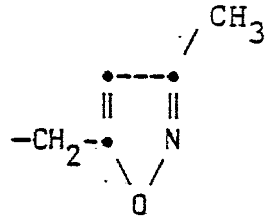

C: 4-Chlorbenzyl

D: 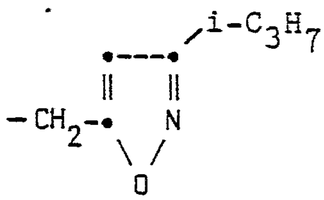

E: 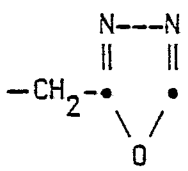

F: 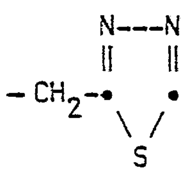

T: 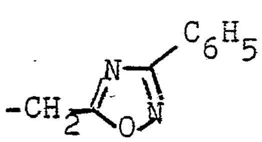

G: $-CH_2-CCl=CCl_2$

I: 4-Fluorbenzyl

K: 2,4-Dichlorbenzyl

L: 3-Fluorbenzyl

M: 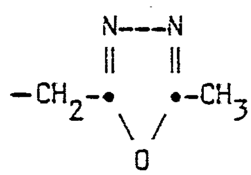

N: 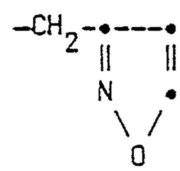

R: $-CH_2CH_2-CCl=CCl_2$

| | X | Y | Z | $R^1$ | $R^2$ | $R^3$ | Salz | $Fp/n_D$ |
|---|---|---|---|---|---|---|---|---|
| 3 | H | H | H | $CH_3$ | H | $CO_2CH_3$ | - | 73- 77 |
| 4 | " | " | " | $C_2H_5$ | H | $CO_2CH_3$ | - | 75- 78 |
| 5 | " | " | " | A | H | $CO_2CH_3$ | - | 180-182 |
| 6 | H | H | F | $CH_3$ | $CH_3$ | H | HBr | |
| 7 | " | " | " | " | " | " | - | 48- 50 |
| 8 | " | " | " | Benzyl | " | " | - | |
| 9 | " | " | " | B | $CH_3$ | H | - | |
| 10 | " | " | " | $CH_3$ | H | $CO_2CH_3$ | - | |
| 11 | H | F | H | $CH_3$ | $CH_3$ | H | - | 1.5980 |
| 12 | " | " | " | " | " | " | HJ | 170-173 |
| 13 | " | " | " | Benzyl | $CH_3$ | " | - | 1.6208 |
| 14 | " | " | " | " | " | " | HBr | |
| 15 | " | " | " | A | $CH_3$ | " | - | 61- 63 |
| 16 | " | " | " | " | " | " | HCl | 65- 66 |
| 17 | " | " | " | " | " | " | HBr | |
| 18 | " | " | " | C | $CH_3$ | " | - | 1.6190 |
| 19 | " | " | " | " | " | " | HBr | 159-160 |
| 20 | " | " | " | B | " | " | - | 58- 60 |
| 21 | " | " | " | " | " | " | HCl | 139-141 |
| 22 | " | " | " | D | " | " | - | |
| 23 | " | " | " | E | " | " | - | |
| 24 | " | " | " | F | " | " | - | |
| 25 | " | " | " | " | " | " | HCl | |
| 26 | " | " | " | G | $CH_3$ | " | HCl | 1.6211 |
| 27 | " | " | " | " | " | - | - | 1.6103 |
| 28 | " | " | " | $CH_3$ | H | - | | |
| 29 | F | H | H | $CH_3$ | $CH_3$ | H | - | |
| 30 | " | " | " | Benzyl | $CH_3$ | H | HBr | |
| 31 | " | " | " | " | " | " | - | |

| | X | Y | Z | $R^1$ | $R^2$ | $R^3$ | Salz | $Fp/n_D$ |
|---|---|---|---|---|---|---|---|---|
| 32 | F | H | H | R | $CH_3$ | H | HCl | |
| 33 | " | " | " | " | " | " | - | |
| 34 | " | " | " | B | $CH_3$ | H | HCl | |
| 35 | " | " | " | " | " | " | - | |
| 36 | " | " | " | $CH_3$ | H | $CO_2CH_3$ | - | |
| 37 | H | Cl | H | $CH_3$ | $CH_3$ | H | HJ | |
| 38 | " | " | " | " | " | " | - | |
| 39 | " | " | " | R | $CH_3$ | " | HCl | |
| 40 | " | " | " | " | " | " | - | |
| 41 | " | " | " | B | $CH_3$ | " | HCl | |
| 42 | " | " | " | " | " | " | - | |
| 43 | " | " | " | $CH_3$ | H | $CO_2CH_3$ | - | |
| 44 | H | H | Cl | $CH_3$ | H | $CO_2CH_3$ | - | 81- 83 |
| 45 | " | " | " | D | $CH_3$ | H | HCl | |
| 46 | " | " | " | " | " | " | - | |
| 47 | " | " | " | $C_2H_5$ | $CH_3$ | H | - | 1.5962 |
| 48 | " | " | " | Benzyl | $CH_3$ | H | HBr | 1.6301 |
| 49 | " | " | " | " | " | " | - | 1.6255 |
| 50 | H | H | Cl | I | $CH_3$ | H | HBr | 159-160 |
| 51 | " | " | " | " | " | " | - | 57- 58 |
| 52 | " | " | " | A | $CH_3$ | H | HCl | 183-185 |
| 53 | " | " | " | " | " | " | - | 80- 82 |
| 54 | " | " | " | K | " | " | HBr | 184-186 |
| 55 | " | " | " | " | " | " | - | 1.6319 |
| 56 | " | " | " | L | " | " | HBr | 142-145 |
| 57 | " | " | " | " | " | " | - | 1.6229 |
| 58 | " | " | " | Allyl | " | " | HBr | 1.6130 |
| 59 | " | " | " | " | " | " | - | 1.6190 |
| 60 | " | " | " | G | " | " | HCl | 167-172 |
| 61 | " | " | " | G | " | " | - | 1.6246 |
| 62 | H | Cl | H | $CH_3$ | $CH_3$ | H | - | |

| | X | Y | Z | $R^1$ | $R^2$ | $R^3$ | Salz | Fp/$n_D$ |
|---|---|---|---|---|---|---|---|---|
| 63 | H | Cl | H | Benzyl | $CH_3$ | H | - | |
| 64 | " | " | " | B | $CH_3$ | H | HCl | |
| 65 | " | " | " | " | " | " | - | |
| 66 | " | " | " | R | " | " | HCl | |
| 67 | " | " | " | " | " | " | - | |
| 68 | H | H | $CH_3$ | $CH_3$ | $CH_3$ | H | - | 65- 68 |
| 69 | " | " | " | " | " | " | HJ | 175-178 |
| 70 | " | " | " | $C_2H_5$ | " | " | - | 1.6306 |
| 71 | " | " | " | Benzyl | " | " | HBr | 85- 88 |
| 72 | " | " | " | " | " | " | - | 70- 72 |
| 73 | " | " | " | D | " | " | - | |
| 74 | " | " | " | R | " | " | HCl | |
| 75 | " | " | " | " | " | " | - | |
| 76 | H | H | i-$C_3H_7$ | $CH_3$ | H | $CO_2CH_3$ | - | 111-113 |
| 77 | " | " | " | $CH_3$ | $CH_3$ | H | - | 73- 74 |
| 78 | " | " | " | " | " | " | HJ | 183-185 |
| 79 | " | " | " | B | $CH_3$ | H | HCl | |
| 80 | " | " | " | " | " | " | - | |
| 81 | " | " | " | R | H | H | - | |
| 82 | H | $CF_3$ | H | $CH_3$ | $CH_3$ | H | HJ | 122-123 |
| 83 | " | " | " | " | " | " | - | 1.6189 |
| 84 | " | " | " | R | " | " | HCl | |
| 85 | " | " | " | B | " | " | HCl | |
| 86 | " | " | " | " | " | " | - | |
| 87 | H | Cl | Cl | $CH_3$ | $CH_3$ | H | HJ | 168-170 |
| 88 | " | " | " | " | " | " | - | 1.6301 |
| 89 | " | " | " | " | H | $CO_2CH_3$ | - | 91- 92 |
| 90 | " | " | " | Benzyl | " | " | | 1.6035 |
| 91 | " | " | " | B | " | " | - | 70- 75 |
| 92 | " | " | " | Benzyl | $CH_3$ | H | HBr | 189-191 |
| 93 | " | " | " | B | $CH_3$ | H | HCl | 1.6205 |
| 124 | " | " | " | T | $CH_3$ | H | - | 84-87 |

0075205

| | X | Y | Z | $R^1$ | $R^2$ | $R^3$ | Salz | $Fp/n_D$ |
|---|---|---|---|---|---|---|---|---|
| 94 | H | Cl | Cl | B | $i-C_3H_7$ | H | HCl | |
| 95 | " | " | " | " | $CH_3$ | " | - | 110-114 |
| 96 | " | " | " | " | $i-C_3H_7$ | " | HCl | |
| 97 | " | " | " | B | $CH_3$ | " | HCl | |
| 97 | " | " | " | B | $CH_3$ | " | HCl | |
| 98 | " | " | " | " | " | " | - | |
| 99 | " | " | " | D | " | " | HCl | viskos |
| 100 | " | " | " | " | " | " | - | viskos |
| 101 | " | " | " | F | " | " | - | |
| 102 | " | " | " | M | " | " | - | |
| 103 | " | " | " | Benzyl | " | " | HBr | |
| 104 | " | " | " | " | " | " | - | |
| 105 | H | Cl | $OCH_3$ | $CH_3$ | $CH_3$ | H | - | |
| 106 | " | " | " | " | " | " | HJ | |
| 107 | H | " | " | " | H | $CO_2CH_3$ | - | |
| 108 | " | " | " | B | $CH_3$ | H | HCl | |
| 109 | " | " | " | " | " | " | - | |
| 110 | " | " | " | R | " | " | - | |
| 111 | H | $CF_3$ | H | $CH_3$ | $CH_3$ | H | - | |
| 112 | " | " | " | " | " | " | HJ | |
| 113 | " | " | " | B | $CH_3$ | H | HCl | |
| 114 | " | " | " | " | " | " | - | |
| 115 | " | " | " | R | $CH_3$ | H | - | |
| 116 | " | " | " | $CH_3$ | H | $CO_2CH_3$ | - | |
| 117 | " | Cl | Cl | N | $CH_3$ | H | HCl | viskos |
| 118 | " | " | " | " | " | " | HBr | |
| 119 | " | " | " | " | " | " | - | viskos |
| 120 | H | H | Cl | 2-Phenoxy-ethyl | $CH_3$ | H | HBr | Öl |
| 121 | H | Cl | Cl | " | $CH_3$ | H | HBr | Öl |
| 122 | H | Cl | Cl | " | $CH_3$ | H | - | Öl |
| 123 | H | Cl | Cl | T | $CH_3$ | H | HCl | 154-156 |

Die Wirkung der Isothioharnstoffe der Formel I bzw. der sie enthaltenden herbiziden Mittel auf das Wachstum von erwünschten und unerwünschten Pflanzen wird durch Gewächshausversuche gezeigt:

Als Kulturgefäße dienten Plastikblumentöpfe mit 300 $cm^3$ Inhalt und lehmigem Sand mit etwa 1,5 % Humus als Substrat. Bei dem für die Nachauflaufbehandlung angezogenen Reis wurde zusätzlich Torfmull beigemischt, um ein einwandfreies Wachstum zu gewährleisten. Die Samen der Testpflanzen säte man nach Arten getrennt flach ein. Unmittelbar danach erfolgte bei Vorauflaufbehandlung das Aufbringen der Wirkstoffe auf die Erdoberfläche. Sie wurden hierbei in Wasser als Verteilungsmittel suspendiert oder emulgiert und mittels fein verteilender Düsen gespritzt. Bei der Applikationsmethode handelt es sich je nach Wirkstoff um 3,0 kg/ha. Nach dem Aufbringen der Mittel wurden die Gefäße leicht beregnet, um Keimung und Wachstum in Gang zu bringen. Danach deckte man die Gefäße mit durchsichtigen Plastikhauben ab, bis die Pflanzen angewachsen waren. Diese Abdeckung bewirkte ein gleichmäßiges Keimen der Testpflanzen, sofern dies nicht durch die Wirkstoffe beeinträchtigt wurde.

Zum Zwecke der Nachauflaufbehandlung zog man die Testpflanzen je nach Wuchsform erst bis zu einer Wuchshöhe von 3 bis 15 cm an und behandelte sie danach. Zur Nachauflaufbehandlung wurden entweder direkt gesäte und in den gleichen Gefäßen aufgewachsene Pflanzen ausgewählt, oder sie wurden erst als Keimpflanzen getrennt angezogen und einige Tage vor der Behandlung in die Versuchsgefäße verpflanzt. Die Aufwandmengen für die Nachauflaufbehandlung waren ebenfalls je nach Verbindung unterschiedlich und betrugen 0,5, 1,0 2,0 oder 3,0 kg/ha Wirkstoff. Die Abdeckung unterblieb bei der Nachauflaufbehandlung.

Die Versuchsgefäße wurden im Gewächshaus aufgestellt, wobei für wärmeliebende Arten wärmere Bereiche (20 bis 35°C) und für solche gemäßigter Klimate 15 bis 25°C bevorzugt wurden. Die Versuchsperiode erstreckte sich über 3 bis 4 Wochen.

Während dieser Zeit wurden die Pflanzen gepflegt und ihre Reaktion auf die einzelnen Behandlungen ausgewertet. Bewertet wurde nach einer Skala von 0 bis 100. Dabei bedeutet 0 keine Schädigung oder normaler Ablauf und 100 kein Aufgang der Pflanzen bzw. völlige Zerstörung zumindest der oberirdischen Teile.

Die Testpflanzen setzen sich aus folgenden Pflanzenarten zusammen:

| | |
|---|---|
| Amaranthus retroflexus | (zurückgekrümmter Fuchsschwanz), |
| Arachis hypogaea | (Erdnüsse), |
| Avena sativa | (Hafer), |
| Cassia tora, | |
| Centaura cyanus | (Kornblume), |
| Chenopodium album | (Weißer Gänsefuß), |
| Datura stramonium | (gemeiner Stechapfel), |
| Gossypium hirsutum | (Baumwolle), |
| Hordeum vulgare | (Gerste), |
| Ipomoea spp. | (Prunkwindearten), |
| Oryza sativa | (Reis), |
| Sesbania exaltata | (Turibaum), |
| Sida spinosa, | |
| Sinapis alba | (Weißer Senf), |
| Solanum nigrum | (Schwarzer Nachtschatten), |
| Triticum aestivum | (Weizen). |

Die Testergebnisse zeigen, daß die Verbindungen bei Vor- und Nachauflaufanwendung eine beachtliche herbizide Wirkung haben und gleichzeitig für verschiedene Kulturpflanzen als selektive Mittel verträglich sind oder aber nur geringfügige Schäden an den Kulturpflanzen verursachen.

Bei der Prüfung auf selektive herbizide Wirkung bei Vorauflaufanwendung zeigen beispielsweise die Verbindungen Nr. 2a, 2b, 13, 16, 20, 21, 88 und 89 eine beachtliche herbizide Wirkung und sind gleichzeitig gut verträglich für Getreide, wie z.B. Hafer.

Bei Nachauflaufanwendung besitzen beispielsweise die Verbindungen Nr. 13, 18, 20, 27, 88, 89, 90, 93, 95, 99, 120, 121 und 122 eine nennenswerte herbizide Aktivität.

Die Verbindungen Nr. 27 und 99 haben bei Nachauflaufanwendung eine beachtliche Wirkung gegen verschiedene breitblättrige unerwünschte Pflanzen, wobei Erdnüsse und Baumwolle nicht oder nur geringfügig geschädigt werden.

Ferner bekämpft die Verbindung Nr. 93 eine Reihe breitblättriger unerwünschter Pflanzen und verhält sich dabei gegenüber verschiedenen Kulturpflanzen, wie z.B. Erdnüssen, Baumwolle, Reis, Gerste und Weizen, völlig bzw. nahezu schadlos selektiv.

Sind gewisse Kulturpflanzen gegenüber den Wirkstoffen etwas empfindlich, so können auch Ausbringungstechniken angewandt werden, bei welchen die herbiziden Mittel mit Hilfe der Spritzgeräte so geleitet werden, daß die Blätter empfindlicher Kulturpflanzen nach Möglichkeit nicht getroffen werden, während sie auf die Blätter darunter

wachsender unerwünschter Pflanzen oder die unbedeckte Bodenfläche gelangen (post-directed, lay-by). In Anbetracht der guten Verträglichkeit und der Vielseitigkeit der Applikationsmethoden können die erfindungsgemäßen Herbizide noch in einer weiteren großen Zahl von Kulturpflanzen zur Beseitigung unerwünschten Pflanzenwuchses eingesetzt werden. Die Aufwandmengen können dabei zwischen 0,1 und 15 kg/ha und mehr schwanken.

Zur Verbreiterung des Wirkungsspektrums und zur Erzielung synergistischer Effekte können die Verbindungen der Formel I Verbindungen mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzofuranderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem ist es nützlich, die neuen Verbindungen allein oder in Kombination mit anderen Herbiziden auch noch mit weiteren Pflanzenschutzmitteln gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die erfindungsgemäßen Mittel können in Form üblicher Formulierungen angewendet werden wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung oder wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel zugesetzt werden können. Als Hilfsstoffe zur Formulierung kommen im wesentlichen in Frage Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin), Dimethylformamid und Wasser; feste Trägerstoffe wie natürliche Gesteinsmehle (z.B. Kaoline, Tonerden, Talkum, Kreide) und synthetische Ge--steinsmehle (z.B. hochdisperse Kieselsäure, Silikate); Emulgiermittel oder sonstige oberflächenaktive Mittel, wie nichtionogene und anionische Emulgatoren (z.B. Polyoxyethylen-Fettalkohol-Ether, Alkylsulfonate) und Dispergiermittel wie Lignin, Sulfitablaugen und Methylcellulose. Bevorzugt ist die Anwendung der erfindungsgemäßen Verbindungen in wäßriger Lösung, gegebenenfalls unter Zusatz von mit Wasser mischbaren organischen Lösungsmitteln wie Methanol oder anderen niederen Alkoholen, Aceton, Dimethylformamid oder N-Methylpyrrolidin. Die Formulierungen enthalten im allgemeinen zwischen 0,1 und 95 Gewichtsprozent Wirkstoff, vorzugsweise zwischen 0,5 und 90.

Die Formulierungen bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise im Vorauflaufverfahren, im Nachauflaufverfahren oder als Beizmittel.

Beispiele für Formulierungen sind:

I. 20 Gewichtsteile der Verbindung des Beispiels 13 werden in 3 Gewichtsteilen des Natriumsalzes der Diisobutylnaphthalin-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

II. 3 Gewichtsteile der Verbindung des Beispiels 27 werden mit 97 Gewichtsteilen feinteiligem Kaolin innig vermischt. Man erhält auf diese Weise ein Stäubemittel, das 3 Gew.% des Wirkstoffs enthält.

III. 30 Gewichtsteile der Verbindung des Beispiels 26 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

IV. 40 Gewichtsteile der Verbindung des Beispiels 21 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gew.% Wirkstoff enthält.

V. 20 Teile der Verbindung des Beispiels 15 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

VI. Man vermischt 90 Gewichtsteile der Verbindung des Beispiels 88 mit 10 Gewichtsteilen N-Methyl--pyrrolidon und erhält eine Lösung, die zur Anwendung in Form kleinster Tropfen geeignet ist.

VII. 20 Gewichtsteile der Verbindung des Beispiels 29 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure--N-monoethanolamid, 5 Gewichtsteilen Calciumsalz der Dodecylbenzolsulfonsäure und 5 Gewichtsteile des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

VIII. 20 Gewichtsteile der Verbindung des Beispiels 13 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IX. 10 Gewichtsteile der Verbindung des Beispiels 93, 20 Gewichtsteile Polyoxyethylensorbitan-monolaurat, 20 Gewichtsteile Methanol und 50 Gewichtsteile Wasser werden zu einer Lösung verrührt, die 10 Gew.% des Wirkstoffs enthält. Durch Hinzufügen von weiterem Wasser können stärker verdünnte Lösungen hergestellt werden.

## Patentansprüche

1. Isothioharnstoffe der Formel

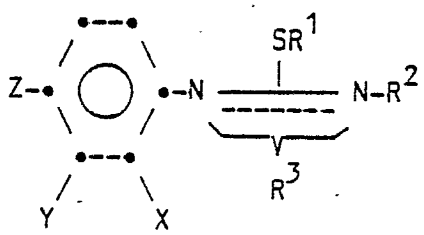

(I).

und ihre Salze,
wobei die Formel I die Isomeren der Formel Ia

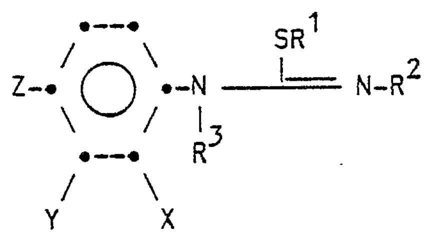

(Ia)

und der Formel Ib

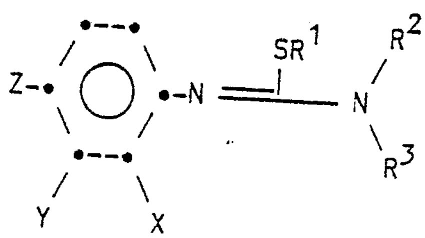

(Ib)

kennzeichnet, worin
X, Y und Z gleich oder verschieden sind und Wasserstoff, Halogen, $C_{1-4}$-Alkyl, $C_{1-4}$-Alkoxy, $C_{1-4}$-Halogenalkyl, $C_{1-4}$Halogenalkoxy, $C_{1-4}$-Alkylthio,
$R^1$ $C_{1-7}$-Alkyl oder $C_{3-7}$-Cycloalkyl, $C_{1-4}$-Halogenalkyl, $C_{2-8}$-Alkylthioalkyl, Benzyl, substituiertes Benzyl, $C_{2-4}$-Alkenyl, $C_{2-4}$-Halogenalkenyl, $C_{2-4}$-Alkinyl, einen heterocyclischen Rest tragendes $C_1$-$C_4$-Alkyl, Aryloxy-$C_{1-4}$-alkyl,

$R^2$ Wasserstoff, $C_{1-7}$-Alkyl oder $C_{3-7}$-Cycloalkyl,
$R^3$ Wasserstoff oder die Gruppe $-CO-OR^4$, worin $R^4$ $C_{1-3}$Alkyl ist,
bedeuten, mit der Maßgabe, daß wenn Z Chlor, Y Wasserstoff, X Wasserstoff, $R^2$ Alkyl und $R^3$ Wasserstoff sind, $R^1$ nicht Alkyl ist und $R^2$ und $R^3$ nicht gleichzeitig Wasserstoff bedeuten,
und Salze dieser Isothioharnstoffe.

2. Isothioharnstoffe der Formel I gemäß Anspruch 1, dadurch gekennzeichnet, daß X Wasserstoff, Y Wasserstoff, Fluor oder Chlor, Z Wasserstoff oder Chlor, $R^1$ eine durch einen gegebenenfalls methylierten Isoxazolyl-, Thiadiazolyl- oder Oxadiazolylrest oder einen gegebenenfalls chlorierten Phenylrest oder Trichlorethylenrest substituierte Methylgruppe, $R^2$ eine Methyl- oder Acetoxygruppe und $R^3$ Wasserstoff bedeuten.

3. Isothioharnstoffe der Formel I gemäß Anspruch 2, dadurch gekennzeichnet, daß $R^1$ den 3-Methyl-isoxazol-5-yl-methylrest bedeutet.

4. Herbizid, enthaltend eine Verbindung der Formel I gemäß Anspruch 1.

5. Herbizid, enthaltend inerte Zusatzstoffe und einen Isothioharnstoff der Formel

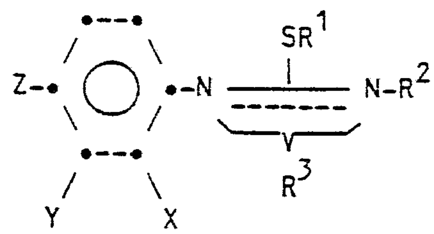 (I)

und ihre Salze,
wobei die Formel I die Isomeren der Formel Ia

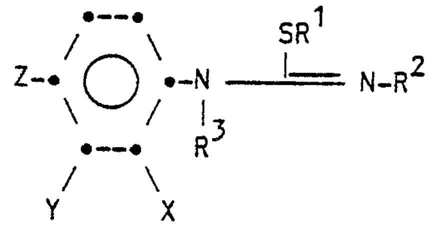 (Ia)

und der Formel Ib

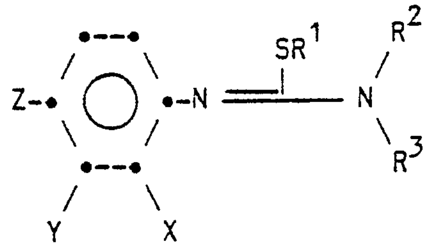 (Ib)

kennzeichnet, worin

X, Y und Z gleich oder verschieden sind und Wasserstoff, Halogen, $C_{1-4}$-Alkyl, $C_{1-4}$-Alkoxy, $C_{1-4}$-Halogenalkyl, $C_{1-4}$Halogenalkoxy, $C_{1-4}$-Alkylthio,

$R^1$ $C_{1-7}$-Alkyl oder $C_{3-7}$-Cycloalkyl, $C_{1-4}$-Halogenalkyl, $C_{2-8}$-Alkylthioalkyl, Benzyl, substituiertes Benzyl, $C_{2-4}$-Alkenyl, $C_{2-4}$-Halogenalkenyl, $C_{2-4}$-Alkinyl, einen heterocyclischen Rest tragendes $C_{1-4}$Alkyl, Aryloxy-$C_{1-4}$-alkyl,

$R^2$ Wasserstoff, $C_{1-7}$-Alkyl oder $C_{3-7}$-Cycloalkyl,

$R^3$ Wasserstoff oder die Gruppe $-CO-OR^4$, worin $R^4$ $C_{1-3}$Alkyl ist,

bedeuten, mit der Maßgabe, daß wenn Z Chlor, Y Wasserstoff, X Wasserstoff, $R^2$ Alkyl und $R^3$ Wasserstoff sind, $R^1$ nicht Alkyl ist und $R^2$ und $R^3$ nicht gleichzeitig Wasserstoff bedeuten.

6. Verfahren zur Bekämpfung unerwünschten Pflanzenwuchses, dadurch gekennzeichnet, daß man die Pflanzen und/oder ihren Standort mit einer herbizid wirksamen Menge einer Verbindung der Formel I gemäß Anspruch 1 behandelt.

7. Verfahren zur Herstellung der Verbindungen der Formel I gemäß Anspruch 1, dadurch gekennzeichnet, daß man

a) eine Halogenalkyl-Verbindung der Formel

$$\text{Hal-R}^1 \qquad (II),$$

worin $R^1$ die im Anspruch 1 genannte Bedeutung hat und Hal Halogen bedeutet, mit einem Thioharnstoff der Formel

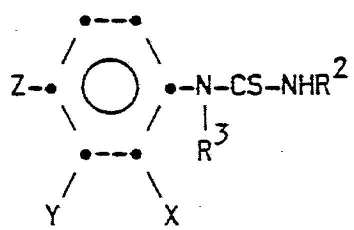

(III),

worin X, Y, Z, $R^2$ und $R^3$ die im Anspruch 1 genannten Bedeutungen haben, oder

b) einen Isothioharnstoff der Formel

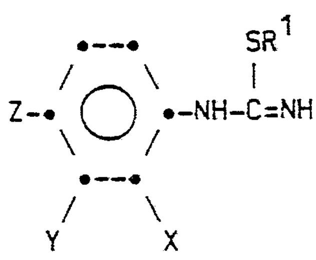

(IV),

worin X, Y, Z und $R^1$ die im Anspruch 1 genannten Bedeutungen haben, mit einem Chlorameisensäureester der Formel

Hal-CO-OR$^4$ (V),

worin $R^4$ die im Anspruch 1 angegebene Bedeutung hat und Hal für Halogen steht, umsetzt, und die so erhaltenen Verbindungen gegebenenfalls anschließend in ihre Salze überführt.